# EUROPEAN PATENT SPECIFICATION

(11) **EP 2 588 038 B1**
(45) Date of publication and mention of the grant of the patent: **19.03.2014**
(21) Application number: 11728043.8
(22) Date of filing: 14.06.2011
(51) Int. Cl.: A61F 2/46

(54) **INSTRUMENT FOR USE IN MANIPULATING AN IMPLANT COMPONENT**
INSTRUMENT ZUR VERWENDUNG BEI DER MANIPULATION EINER IMPLANTATKOMPONENTE
INSTRUMENT DESTINÉ À ÊTRE UTILISÉ DANS LA MANIPULATION D'UN COMPOSANT D'IMPLANT

(30) Priority: 11.10.2010 GB 201017041; 01.07.2010 GB 201011097
(43) Date of publication of application: 08.05.2013
(73) Proprietor: Finsbury (Development) Limited, Leatherhead, Surrey KT22 7BA (GB)
(72) Inventor: STACEY, Thomas, Leeds West Yorkshire LS11 8DT (GB)
(74) Representative: Belcher, Simon James
(86) International application number: PCT/GB2011/051110
(87) International publication number: WO 2012/001385

(56) References cited:
- WO-A2-2008/016312
- US-A- 5 059 196
- US-A- 5 417 693
- US-A- 5 928 287

## Description

This invention relates to an instrument for use in manipulating an implant component.

A component that is to be implanted in a patient must be manipulated prior to and during the implantation procedure. It can be important to grip the component securely. It can be important to grip the component in such a way that features of the component are not damaged.

The invention is applicable to the manipulation of components of orthopaedic joint prostheses. It is common for such components to have significant forces applied to them during implantation. They can have highly polished bearing surfaces and other features which should be protected from damage.

A knee joint prosthesis includes a femoral component and a tibial component. It is common to manipulate the femoral component using an instrument in which the component is held securely. An impaction force can be applied to the femoral component through the instrument. It is important that the polished bearing surface of the component is not damaged by the instrument during manipulation and application of the impaction force.

US-5732992 discloses a tool for engaging the femoral component of a knee joint prosthesis which has medial and lateral arms mounted on a threaded shaft which has a right-handed thread at one end to engage one of the arms and a left-handed thread at the opposite end to engage the other arm. The arms can be drawn together by rotation of the shaft. This causes fingers on the arms to grip the medial and lateral edges respectively of the distal portion of the femoral component. The tool includes an impact base which acts against the distal portion of the polished bearing surface of the femoral component. Continued rotation of the threaded shaft causes the arms to pivot so that they press against the impact base, urging it towards the bearing surface of the component. The component is then held between the fingers and the impact base.

US-5928287 discloses an instrument for use in implanting an acetabular cup. The head of the instrument is provided by a cylindrical block which can fit inside the cup. The head has a pair of parallel bores within it, spaced apart from the midline of the head. Each of the bores has a slide piece which is urged outwardly by means of a spring. The faces of the slide pieces which face the midline of the head are designed as toothed racks, and they engage a centrally arranged pinion which is provided at the end of a shaft. When the pinion is rotated, the slide pieces are made to slide into or out of the bores, depending on the direction of rotation of the pinion. Each of the slide pieces has a projection at its outward end which can be received in a bore in the end face of the cup.

The present invention provides an instrument for use in manipulating an implant component in which a bed for engaging a face of the implant component is provided on a slidable shaft which is mounted in the housing so that it can slide along a shaft axis in the housing, and in which the instrument includes a ramp surface and a follower which can move along the ramp surface with one of the ramp surface and the follower being fixed relative to the shaft so that the position of the shaft relative to the housing along the shaft axis is determined by the position of the follower on the ramp surface. The position of the follower on the ramp surface varies according to the positions of the first and second slidable arms relative to the housing.

The instrument which is provided by the invention is defined in claim 1.

The instrument of the invention has the advantage that the relationship between the movement of the first and second arms and the movement of the shaft can be defined by the ramp surface and its engagement by the follower. This can facilitate secure attachment of the instrument to the implant component.

In addition, the separation of the sliding movement of the shaft from the movement of the arms means that the movement of the arms can be translation only. This allows the instrument to be used to engage implant components with a wide range of sizes, limited only by the length of the paths on which the arms slide. This is in contrast to instruments in which movement of arms pivot to engage an implant involves pivoting of the arms.

Preferably, the follower comprises a projection on the shaft which extends in a direction which is generally transverse relative to the shaft axis. For example, the projection can comprise a pin which can be fitted at one end into a bore in the shaft.

It is envisaged that the ramp surface can be provided on the shaft. It can be engaged by a follower which is provided on another component of the instrument, for example on the housing or on one or both of the arms.

The ramp surface can be provided by a groove (or slot) and a follower in the form of a projection can slide in the groove. The groove can be blind or it can be open at its base. An open groove can be preferred for ease of cleaning.

The gear mechanism for the arms ensures that the sliding movement of the first arm in a first direction relative to the housing is accompanied by sliding movement of the second arm through an equal distance in a second direction which is opposite to the first direction. This can be accomplished, for example, using a threaded shaft which is mounted in the housing for rotation about its axis, and which has a right-handed thread at one end to engage one of the arms and a left-handed thread at the opposite end to engage the other arm.

Preferably, the gear mechanism comprises first and second toothed racks which are fixed to the first and second arms respectively. A toothed gear is positioned in the housing between the toothed racks whose teeth engage the teeth on the first and second racks and which is made to rotate in the housing when the arms slide relative to the housing.

The gear mechanism which links the first arm to the second arm can cause the shaft to rotate about the shaft axis when then first arm is made to slide relative to the second arm. Preferably, the gear mechanism comprises first and second toothed racks which are fixed to the first and second arms respectively, and in which the shaft has a toothed gear located between the toothed racks whose teeth engage the teeth on the first and second racks so that the shaft is made to rotate in the housing by the action of the racks on the toothed gear when the arms slide relative to the housing.

The shaft can have a connector for a tool by which the shaft can be driven rotationally relative to the housing. The connector can be provided by a portion of the shaft which has a cross-section which is not circular, for example with one or more flat sides. For example, the connector can be provided at the end of the shaft which is opposite to the end on which the bed is provided. The connector end of the shaft can have one or more flat sides.

The instrument can be driven to engage the implant component by applying rotational drive to the shaft in embodiments in which the shaft is able to rotate in the housing and in which the shaft is linked to the arms so that rotation of the shaft causes the arms to slide relative to the housing. Accordingly, the shaft can be rotated to cause it to slide out of the housing, which at the same time causes the arms to slide in to the housing so that the distance between the fingers on the ends of the arms reduces.

The instrument can be driven to engage the implant component by squeezing the arms inwardly so that the fingers on the ends of the arms tighten on to the implant component. The action of the follower against the ramp surface causes the shaft to move relative to the housing. As discussed elsewhere, the movement of the shaft is a translation relative to the housing. The movement of the shaft will include rotation about its axis in some embodiments. The movement of the shaft can be without rotation about its axis in some embodiments.

The ramp surface which is engaged by the follower can be provided by the housing. For example, it can be preferred for the ramp surface to be provided on a wall of a bore in the housing in which the shaft rotates. Accordingly, the follower can move over the ramp surface as the shaft rotates. The ramp surface can be provided by the wall of an opening which communicates with the bore in the housing. The opening can be blind or it can be extend entirely through the housing between the outside of the housing and the bore.

A ramp surface which is engaged by the follower can be provided on one or more of the arms. The or each ramp surface can be engaged by a follower extending from the shaft as the arm is moved in the housing translationally relative to the housing, and to the shaft in the housing. The ramp surface might be provided on just one of the arms. A first ramp surface might be provided on the first arm and a second ramp surface might be provided on the second arm. The shaft can then have first and second projections for engaging the first and second ramp surfaces, respectively.

A follower might be provided on one or more of the arms, extending from its arm towards the ramp surface. A ramp surface might be provided on the shaft which can be engaged by the follower which is provided on the arm. For example, a ramp surface might be provided by a groove which is cut in the side of the shaft. A projection might be provided on just one of the arms. A first projection might be provided on the first arm and a second projection might be provided on the second arm. The shaft can then have first and second ramp surfaces for engagement by the first and second projections, respectively.

Preferably, the ramp surface defines a plurality of steps which correspond to predetermined positions of the shaft relative to the housing. The dependence of the position of the follower on the ramp surface on the positions of the first and second arms relative to the housing means that the shaft (with the bed) can move between a plurality of predetermined positions relative to the housing as the arms are moved continuously relative to the housing. This facilitates gripping of different sizes of implant components which have different sizes, in which each different size of implant component has a known relationship between (a) the distance between the two points on the edges of the component where it is engaged by the fingers on the first and second arms, and (b) the distance between a line joining the points on the edges which are engaged by the fingers and the bed.

It will generally be the case that an implant component will have defined points at which it will be engaged by the fingers on the arms of the instrument. For example, the implant component might have recesses in which the fingers can be received.

When the implant component is a femoral component of a knee joint prosthesis, the fingers can fit into recesses on the medial and lateral edges adjacent to the part of the bearing surface which fits against the distal end of the femur. The bed will then fit against the bearing surface of the femoral component between the recesses.

It is common for femoral components of a knee joint prosthesis to be provided in different sizes so as to optimise fit according to a patient's anatomy. The different sizes are characterised by different relationships between the medial-lateral width and the thickness of the component in the distal region. These dimensions correspond to (a) the distance between the two points on the medial and lateral edges of the component where it is engaged by the fingers on the first and second arms, and (b) the distance between a line joining the points on the edges which are engaged by the fingers and the bed. The instrument of the present invention can provide a good fit between the instrument and the implant component in more than one of the sizes by providing the ramp surface with a suitable profile, with discrete steps on the ramp surface which correspond to the different sizes.

Preferably, the instrument includes a ratchet mechanism which restricts movement of the follower relative to the ramp surface to movement in an engage direction. Accordingly, the engagement between the instrument and the implant component using the fingers on the arms and the bed can become progressively more secure. Preferably, the instrument includes a release mechanism for disengaging the ratchet mechanism to allow movement in an opposite disengage direction.

The ratchet mechanism can be provided by a ratchet projection which engages with a series of ratchet recesses. The ratchet projection can be biassed outwardly towards a position in which it engages the recesses. The spring or other source of a biassing force can be deformed so that the projection can pass between adjacent recesses. For example, each of the recesses can be defined by an inclined face which causes the ratchet projection to be displaced as it passes over the face into an adjacent recess. When the shaft is able to rotate in a bore in the housing, the ratchet mechanism can be provided by a ratchet projection mounted on one of the internal wall of the bore in the housing and the shaft, and a series of ratchet recesses provided on the other of the internal wall of the bore in the housing and the shaft. Preferably, the ratchet projection is provided on the shaft and a series of ratchet recesses are provided in the wall of the bore in the housing.

Preferably, a release mechanism for disengaging the ratchet mechanism comprises a displacement tool which can contact the ratchet projection and cause it to be retracted so that it is no longer received in any of the recesses. In an embodiment of the instrument in which the shaft is able to rotate in a bore in the housing, this can release the shaft to rotate in the bore so that the bed is retracted towards the instrument housing. The functional connection between the shaft and the arms means that the arms can move so that they are disengaged from the implant component.

Preferably, each of the arms is biassed to slide outwardly relative to the housing.

Preferably the portion of the bed or of the fingers or each of them which engages the implant component is formed so that the risk of damage to the implant component is minimised. When the fingers do not engage a bearing surface, it can be appropriate for many applications for them to be made from metal. They should be shaped so that neither the fingers nor the implant component will be deformed as a result of the engagement of the fingers with the implant component. For example the surface of the fingers which engages the implant component might be rounded (in a convex sense).

In many applications, it will be preferred that the shape of the bed should correspond to the shape of the face of the implant component which it contacts when the instrument engages the component. When the face is a convex bearing surface, the bed should have a correspond concave surface in which the convex surface of the implant component can be received. The material of the bed where it contacts the face of the implant component should be selected so that it will not cause the implant component to be damaged when the instrument engages the component. When the implant component comprises a metal, the bed can be formed from a polymeric material. An example of a suitable material is an ultrahigh molecular weight polyethylene. Suitable materials for use in surgical instruments which are to contact bearing surfaces of implant components are known. A suitable material should, when required, be able to transmit impaction forces to the implant component without significant undesirable deformation.

Preferably, the bed is mounted on the shaft so that its orientation can change so as to fit optimally to the implant component.

It will generally be preferred to make components of the instrument of the invention from a metallic material. Suitable metallic materials are known for the manufacture of surgical instruments. Such materials include certain stainless steels.

Components of the housing such as the front and back walls, the first and second side walls, the base wall, the ramp plate can be provided as separate components which might be fastened together to assemble the housing. They might be fastened together by means of fasteners such as threaded fasteners or they might be fastened together permanently by techniques such as welding. It can be preferred however that the housing be formed from a monolithic block by appropriate machining techniques.

Embodiments of the invention are described below by way of example with reference to the accompanying drawings, in which:
Figure 1 is an isometric view of an instrument according to the invention for manipulating a femoral component of a knee joint prosthesis.
Figure 2 is a plan view of the instrument shown in Figure 1.
Figure 3 is a front view of the instrument shown in Figure 1.
Figure 4 is view from below of the instrument shown in Figure 1.
Figure 5 is an exploded view of the instrument shown in Figure 1.
Figure 6 is a sectional elevation through the instrument on the line A-A in Figure 2.
Figure 7 is a transverse sectional elevation through instrument viewed from above on the line B-B in Figure 3.
Figure 8 is a transverse sectional elevation through instrument viewed from below on the line C-C in Figure 3.
Figure 9 is a transverse sectional elevation through instrument viewed from below on the line D-D in Figure 3.
Figure 10 show a femoral component of a knee joint prosthesis
Figure 11 is an isometric view of an assembly which includes a femoral component of a knee joint prosthesis in combination with the instrument shown in Figure 1.
Figure 12 is an isometric view of another embodiment of instrument which can be used to engage a femoral component of a knee joint prosthesis in order to manipulate the prosthesis.

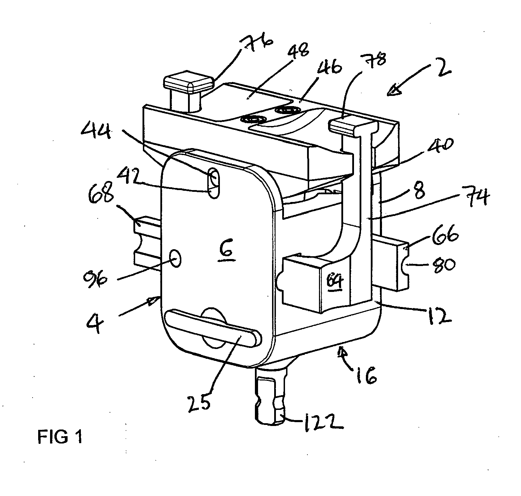

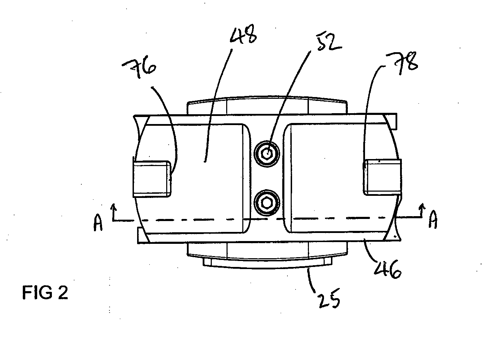

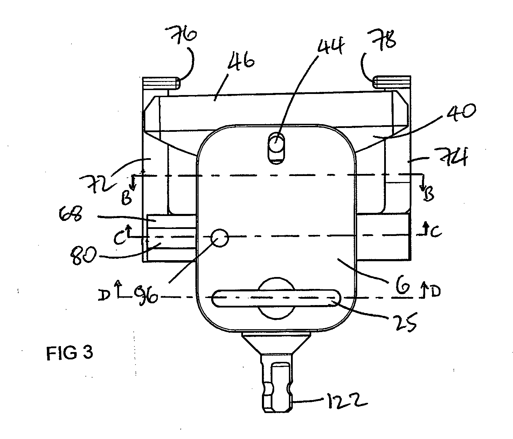

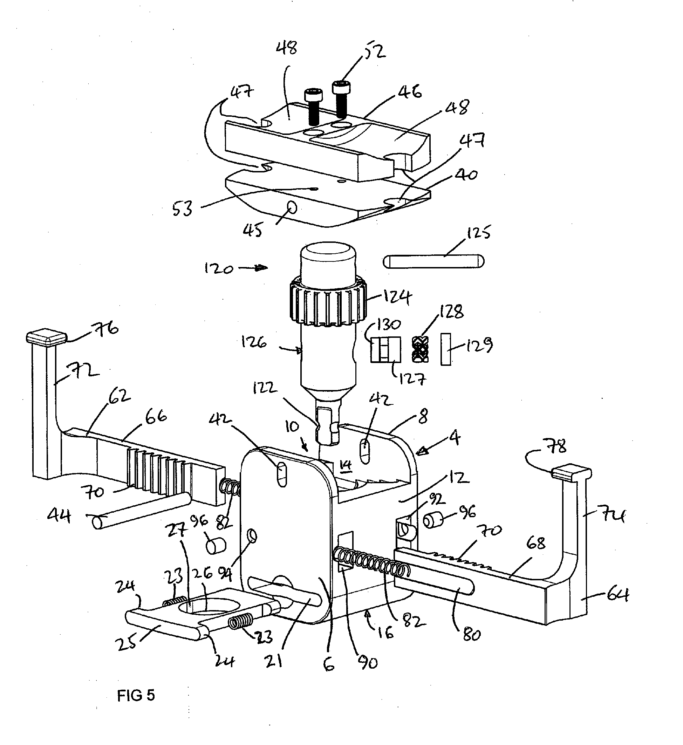

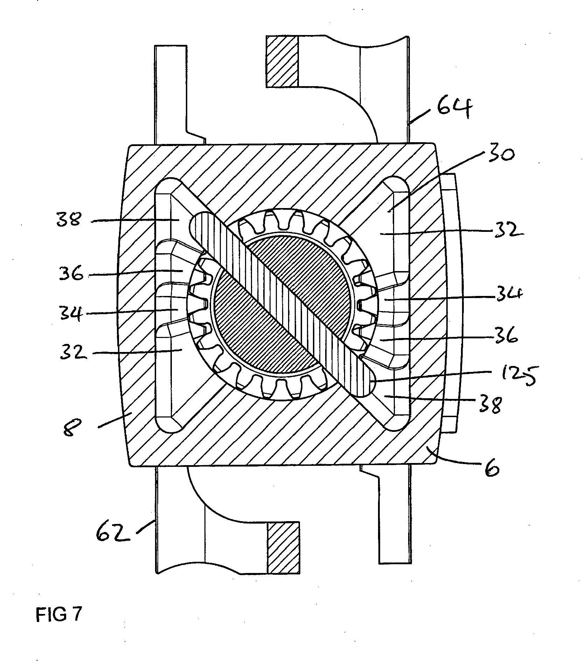

Referring to the drawings, Figures 1 to 9 show an instrument 2 which can be used to apply an implantation force to a femoral component of a knee joint prosthesis. The instrument includes a housing 4 which has front and back walls 6, 8 and first and second side walls 10, 12. The four walls define a hollow space 14 between them. The housing has a base wall 16. The base wall has a central circular aperture 17 formed in it which defines the housing axis. The housing includes a guide plate 18 which has a central circular aperture 19 formed in it. The apertures 17, 19 in the base wall and the guide plate both lie on the housing axis. The top face of the housing which is opposite to the base wall is open. The front and back walls extend past the top of the first and second side walls at the top of the housing to define a channel between them.

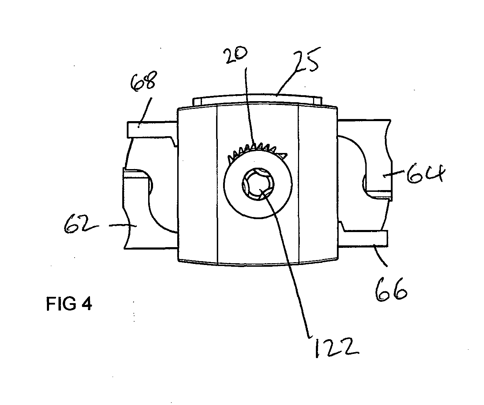

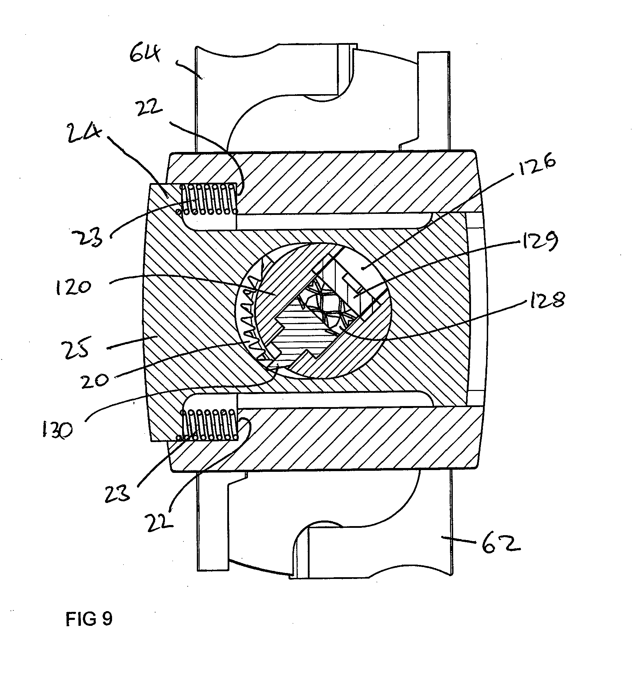

As shown in Figures 4 and 9, the aperture 17 in the base wall has a series of V-shaped recesses 20 cut into it, arranged around a portion of the circumference of the hole.

The housing has a slot 21 formed in it, extending inwardly from the front wall 6. Each of the edges of the slot has a step 22 formed in it.

A release plate 25 is located in the slot 21 so that it can slide within the housing on the top of the base wall 16. The release plate has an outwardly extending lug 24 on each of its edges. A spring 23 is located between each of the lugs 24 on the edges of the release plate and the respective steps 22 in the edges of the slot 21. The springs 23 are compressed between the lugs and the steps when the release plate is forced into the housing in a direction from the front wall towards the back wall, biassing the release plate in the opposite direction. It can be seen in Figure 1 and other Figures that the release plate is protruding from the front wall 6 of the housing. The release plate 25 is generally flush with the front wall when it is pressed into the housing (in such a way as to compress the springs 23).

The release plate has an aperture 26 formed in it. The shape of the aperture is that of an elongated circle, with two circular portions whose centres are spaced apart in a direction which is parallel to the side walls of the housing by a distance (represented by flats 27 on the side walls of the aperture) which is approximately equal to the distance through which the release plate can be displaced inwardly in a direction towards the back wall of the housing. The diameter of each of the circles is approximately equal to the diameter of the circular opening in the base wall of the housing. The width of the aperture in the release plate between the flats 27 at its midpoint is not less than the diameter of the circles.

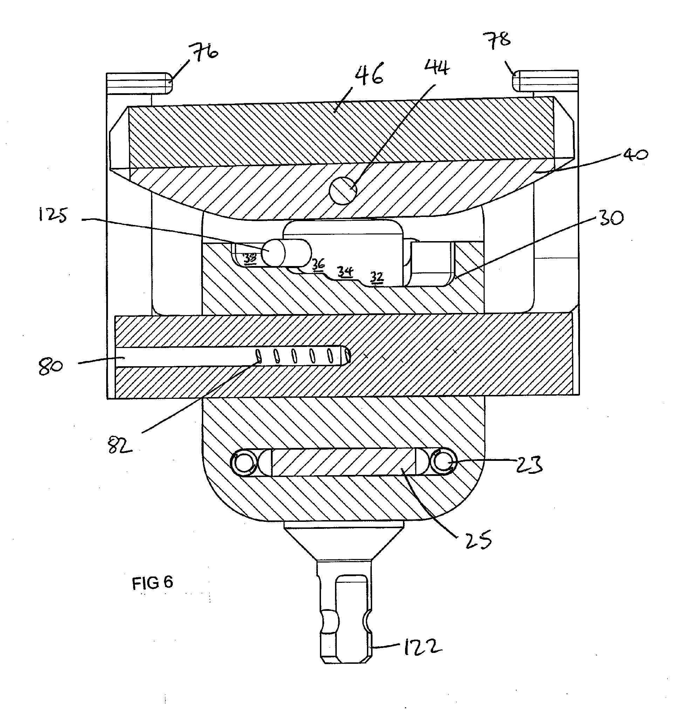

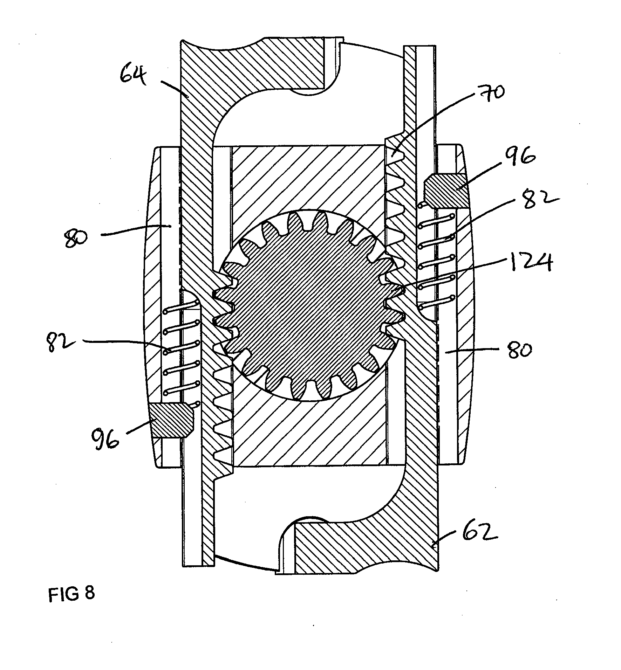

A ramp plate 30 is located within the housing. It extends between the front and back walls and between the first and second side walls. It extends parallel to the base wall between the base wall and the top edges of the side walls. As can be seen in Figures 6 and 8, the ramp plate has a series of upwardly facing ramp surfaces formed in it. The ramp surfaces are provided in two opposed series on the front and back sides of the ramp plate, arranged radially around the axis of the instrument which is defined by the aperture in the base wall. The ramp plate defines a first ramp surface 32 which are located closest to the base wall of the housing. The ramp plate defines second, third and fourth ramp surfaces 34, 36, 38 which are spaced progressively further from the base wall of the housing.

A spindle 120 is located in the hollow space. The spindle 120 has a profiled connector portion 122 at one end whose transverse dimension is less than that of the remainder of the spindle. The outer surface of the spindle provides an annular gear portion 124. The diameter of the spindle between the connector portion and the annular gear portion is such that the spindle is a sliding fit in the aperture 17 in the base wall of the housing. In the embodiment shown in the drawings, the diameter of the gear portion 124 is greater than the diameter of the spindle adjacent to the gear portion. The diameter of the gear portion is a sliding fit in the aperture 19 in the guide plate 18.

The spindle has a first transverse bore extending through it towards its upper end, between the gear portion 124 and the top of the spindle, in which a height locator pin 125 is fixed. As can be seen in Figure 8, the length of the pin 125 is greater than the diameter of the spindle so that the pin extends from the spindle at each of its ends.

The spindle has a second transverse bore 126 extending through it towards its bottom end, between the gear portion 124 and the bottom of the spindle. A ratchet member 127 is positioned so that it can slide in the second transverse bore. The bore contains a ratchet bias spring 128 in the form of a wave spring. The second transverse bore is closed at one end by means of a closure plug 129 which can be held in the bore by means of for example a screw thread or an adhesive. The spring can be compressed between the ratchet member 127 and the plug 129, and biasses the ratchet member in a direction away from the plug. The ratchet member has a tooth 130 on the end which protrudes from the bore 126.

The transverse dimension of the aperture 18 in the base wall of the housing connector portion of the spindle is larger than the diameter of the profiled connector portion 122 of the spindle so that the connector portion of the spindle can extend out of the housing through the aperture when the spindle is located within the housing. In the illustrated embodiment, the diameter of the connector portion of the spindle is 7.95 mm, the diameter of the gear portion is 12.045 mm, the diameter of the remainder of the spindle is 17.5 mm and the diameter of the aperture 18 in the base wall of the housing is 8.75 mm.

The housing includes a generally rectangular pressure plate 40. The width of the pressure plate is less than the width of the channel between the front and back walls of the housing so that the pressure plate can fit between the front and back walls, and slide in the channel in a direction which is parallel to the axis of the instrument defined by the aperture in the base wall. Axial slots 42 are provided in the front and back walls. A locator peg 44 can extend through the slots and through a bore 45 in the pressure plate. The peg can slide in the slots, to define the extent and direction of the sliding movement of the pressure plate relative to the housing.

A bed 46 is provided on the pressure plate 40. The bed is made from a polymeric material such as an ultrahigh molecular weight polyethylene. The bed has two concave recesses 48, 50 formed in it in which the distal portions of the femoral component of a knee joint prosthesis can fit. The use of a polymeric material for the bed which is softer than the material of the bearing surface of the component helps to minimise the risk of damage to the bearing surface when it is contacted by the bed. The bed is fastened to the pressure plate by means of fixing screws 52 which can be received in threaded bores 53 in the pressure plate. A notch 47 is formed in the pressure plate and the bed at each end.

The instrument includes first and second arm members 62, 64. Each of the arm members has a drive limb 66, 68 which has gear teeth 70 on one side face. Each of the arm members has an upstanding arm 72, 74, with an inwardly directed finger 76, 78 at or towards its upper end. A blind channel 80 is formed in the side face of each drive arm which is opposite to the side face on which the gear teeth 70 are provided. A return spring 82 can fit in the channel.

Two pairs of openings 90, 92 are provided in the side walls 10, 12 of the housing. The openings of each pair are aligned so that the drive limb 66 of the first arm member 62 can be positioned so that it extends across the housing, extending through the first pair of openings 90, and so that the drive limb 68 of the second arm member 64 can be positioned so that it extends across the housing, extending through the second pair of openings 92. Each of the arm members can slide through the housing, through the aligned openings.

A stopper hole 94 is provided in each of the front and back walls 6, 8, positioned so that it communicates with the channel 80 its respective drive limb. A stopper plug 96 can be screwed into each of the stopper holes which acts as an end stop for the return spring 82. The return spring can be compressed between the stopper plug and the end of the channel 80 in the drive limb when the arm member is slid through the housing, moving the end of the arm member with the upstanding arm towards the housing. The return spring then biasses the arm member to move in the opposite direction. These features are shown in Figure 9.

The instrument of the invention is assembled by locating the first and second arm members 62, 64 so that they extend through the aligned pairs of openings 90, 92 in the side walls of the housing. The release plate 25 is positioned in the slot 21 in the front wall. The spindle 120 is lowered into the hollow space 14 within the housing so that the connector portion 122 extends through the aperture 17 in the base wall of the housing.

When the spindle is fully seated in the housing, the gear teeth 124 on the spindle the engage the gear teeth 70 on the side faces of the drive limbs 66, 68 of the arm members 62, 64. Furthermore the tooth 130 on the ratchet member 127 engages the V-shaped recesses 20 which are formed in the side of the aperture 17 in the base wall 16 of the housing. The tooth is urged by the spring 128 towards the recesses 20. The engagement of the tooth 130 in successive ones of the recesses is such that the spindle is able to rotate relative to the housing in a clockwise direction (when the instrument is viewed from above) but is prevented from rotating in an anti-clockwise direction.

The length of the tooth 130 on the ratchet member measured in a direction which is parallel to the axis of the instrument is greater than the depth of the recesses 20 measured in the same direction so that the tooth on the ratchet member protrudes from the recesses above the top surface of the aperture 17 in the base wall of the housing. The side wall of the aperture 26 in the release plate 25 therefore acts on the tooth 130 on the ratchet member when the release plate is pressed into the housing, and can displace the tooth from within one of the recesses 20. This allows the spindle to rotate relative to the housing in an anti-clockwise direction (when the instrument is viewed from above)

When the spindle is fully seated in the housing, the height locator pin 125 sits on the ramp plate 30.

The pressure plate 40 is fitted on top of the spindle between the front and back walls 6, 8 of the housing. The locator peg 44 is inserted through the axial slots 42 in the front and back walls and the bore 45 in the pressure plate so that the peg constrains sliding movement of the pressure plate up and down between the front and back walls of the housing. The bed 46 is fastened to the pressure plate by means of the screws 52.

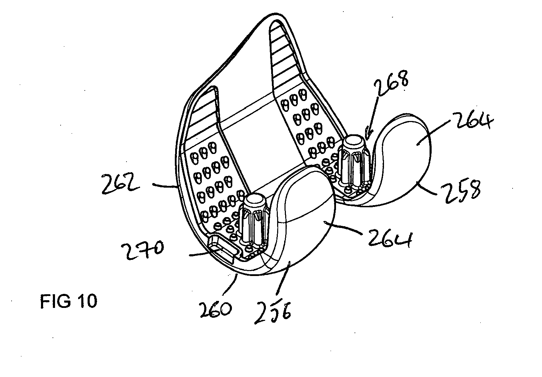

Figure 10 shows a typical femoral component 252 of a knee joint prosthesis. The femoral component has a bone facing surface 254. The distal end of a patient's femur is prepared using conventional techniques so that its shape matches that defined by the bone facing surface of the component, so that the femoral component can be fitted on to the end of the femur.

The component includes lateral and medial condyles 256, 258. The embodiment which is shown in Figure 1 is for fitting to a patient's right knee and the condyles are labelled accordingly. Each of the condyles has a distal portion 260, an anterior portion 262 and a posterior portion 264. The bearing surface 266 of the component is highly polished, extending continuously from the anterior portion through the distal portion to the posterior portion of each of the condyles.

Notches 268, 270 are provided at the lateral and medial edges of the femoral component on the surface which faces away from the bearing surface in the distal portion of the component. The position of the notches is such that they do not interrupt the bearing surface of the component.

Operation of the instrument involves:
1. Releasing the first and second arm members 62, 64 so that they can move outwardly. This is done by pressing the release plate 25 into the slot against the force of the spring 23. The side wall of the openings 26 in the release plate acts against the ratchet member 127 to press it into the second transverse bore 126 in the spindle 120, against the force exerted on the ratchet member by the bias spring 128 in the bore 126. This causes the tooth 130 on the ratchet member to retract from within the V-shaped recesses 20 in the base wall of the housing, allowing the spindle to rotate freely in an anti-clockwise direction (when the instrument is viewed from above) within the housing. The first and second arm members can then move outwardly when the spindle rotates as a result of the engagement between the annular gear portion 124 on the spindle and the teeth 70 on the side faces of the drive limbs 66, 68 of the arm members.
2. Placing the femoral component of a knee joint prosthesis so that the distal condyles sit in the concave recesses 48 in the bed 46.
3. Turning the spindle in a clockwise direction using a drive device which engages the connector portion 122 on the spindle.
4. As a consequence of turning the spindle, causing the arm members to move inwardly, as a result of the engagement between the annular gear portion 124 on the spindle and the teeth 70 on the side faces of the drive limbs 66, 68 of the arm members.
5. As a consequence of turning the spindle, causing the spindle to rise within the housing as a result of the action of the height locator pin 125 on the ramp surfaces 32, 34, 36, 38, which in turn causes the pressure plate 40 and the bed 46 to rise.

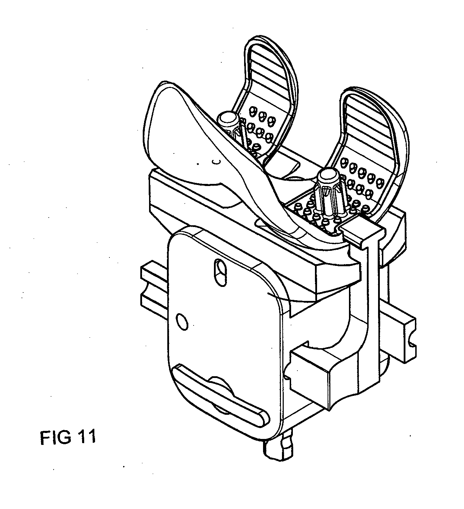

The result of these steps is that the upstanding arms 72, 74 move inwardly towards the medial and lateral edges of the femoral component so that the fingers 76, 78 fit over the edges of the femoral component and engage the notches 268, 270. At the same time, the bed moves in a direction towards the bearing surface of the femoral component. The component then becomes held rigidly between the bed and the fingers. The instrument with the femoral component is shown in this configuration in Figure 11. The instrument can then be used to manipulate the component, in particular when applying an implantation force to the component to fit it to the prepared femur. Provided that an insertion force is not applied to the release plate 25, the bias spring 128 acts on the ratchet member 127 so that the tooth 130 on the ratchet member can fit into the V-shaped recesses in the opening in the base wall of the housing. The ratchet member can then allow rotation of the spindle in the direction which causes the femoral component to be gripped but prevents rotation in the opposite direction. Rotation becomes possible when the release plate is pressed into the housing (as described above).

The instrument of the invention need not use a rotational drive device for actuation. Instead, a clamping force might be initiated by squeezing the two arm members inwardly against the biassing force exerted by the springs 82. This can cause the spindle to rotate as a result of the engagement between the annular gear portion 124 on the spindle and the teeth 70 on the side faces of the drive limbs 66, 68 of the arm members.

The instrument can be geared so that the distance across the device between the fingers 76, 78 on the arm members, and the distance between the bed and a line extending between the fingers, measured in a direction which is aligned with the axis of the housing, ensures that a femoral component is a suitably tight fit in the instrument. The multiple ramp surfaces 32, 34, 36, 38 on the ramp plate 30 can provide for different sizes of femoral component.

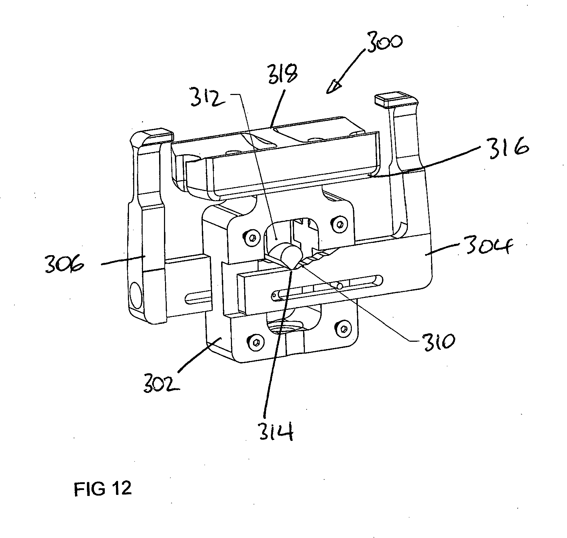

Figure 12 shows an instrument 300 which does not use a rotating spindle. The instrument has a housing 302 with arm members 304, 306 which slide in the housing in a similar manner to the arm member of the instrument which is shown in Figure 1. The arm members have ramp surfaces 308 on their upwardly facing sides.

A central shaft is provided in the housing. A transverse pin 310 extends through the shaft and through vertical slots 312 in the front and back walls of the housing. The pin has a downwardly facing ridge 314.

The instrument includes a pressure plate 316 and a bed 318 which sit on the top of the shaft in a way that is similar to the way in which the pressure plate and the bed sit on top of the spindle in the instrument which is shown in Figure 1.

Operation of the instrument 300 to engage the femoral component of a knee joint prosthesis involves squeezing the arm members 304, 306 inwardly towards the housing. The action of the ridge 314 on the transverse pin 310 against the ramp surfaces 308 on the arm members 304, 306 causes the spindle to rise within the housing. As a result, a femoral component which is placed on the bed becomes gripped between fingers on the end of the arm members and the bed.

The instrument can be used to accommodate femoral components having a range of different sizes. This can be achieved by appropriate selection of the gearing which is used to drive the arms inwardly and shaping of the ramp plate (in particular the shapes of the ramp surfaces 32, 34, 36, and 38) which controls the height of the bed. Examples of the M-L width (measured between the medial and lateral edges of the component at the midpoint of the distal condyles) and the distal depth (measured from the base of the notches in the medial and lateral edges to the distal-most point on the distal condyles) for a range of femoral component sizes are as follows (all dimensions in millimetres):

| Size | M-L width | Depth |
|---|---|---|
| 1 | ≤ 56 | 8 |
| 2 | 57 - 60 | 8 |
| 3 | 61 - 64 | 9 |
| 4 | 65 - 68 | 9 |
| 5 | 68 - 72 | 10 |
| 6 | 72 - 75 | 10 |
| 7 | 76 - 79 | 11 |
| 8 | ≥ 80 | 11 |

## Claims

1. An instrument (2) for use in manipulating an implant component which has a face (266) with two spaced apart edges (268, 270), in which the instrument comprises:
a. a housing (4),
b. first and second arms (62, 64) each having a finger ((76, 78) which can engage the implant component at respective edges of the face, the arms being mounted for sliding movement relative to the housing in which the fingers on the arms move along a common axis,
c. a gear mechanism (70, 124) by which the first and second arms are linked to one another, which ensures that sliding movement of the first arm in a first direction relative to the housing is accompanied by equal sliding movement of the second arm in a second direction which is opposite to the first direction, and
d. a shaft (120) which is mounted in the housing,
**characterised in that** the shaft is mounted in the housing so that it can slide along a shaft axis which is perpendicular to the axis along which the fingers slide, the shaft having a bed (46) at its end for engaging the face of the implant component,
the instrument including a ramp surface (30) and a follower (125) which can move along the ramp surface with one of the ramp surface and the follower being fixed relative to the shaft so that the sliding position of the shaft relative to the housing along the shaft axis is determined by the position of the follower on the ramp surface, in which the position of the follower on the ramp surface varies according to the positions of the first and second arms relative to the housing.

2. An instrument as claimed in claim 1, in which the follower (125) comprises a projection on the shaft (120) which extends in a direction which is generally transverse relative to the shaft axis.

3. An instrument as claimed in claim 2, in which the projection comprises a pin (125).

4. An instrument as claimed in claim 1, in which the gear mechanism comprises first and second toothed racks (70) which are fixed to the first and second arms (62, 64) respectively, and a toothed gear (124) positioned in the housing (4) between the toothed racks whose teeth engage the teeth on the first and second racks and which is made to rotate in the housing when the arms slide relative to the housing.

5. An instrument as claimed in claim 1, in which the gear mechanism (124) which links the first arm (62) to the second arm (64) causes the shaft (120) to rotate about the shaft axis when then first arm is made to slide relative to the second arm.

6. An instrument as claimed in claim 5, in which the gear mechanism comprises first and second toothed racks (70) which are fixed to the first and second arms (62, 64) respectively, and in which the shaft (120) has a toothed gear (124) located between the toothed racks whose teeth engage the teeth on the first and second racks so that the shaft is made to rotate in the housing by the action of the racks on the toothed gear when the arms slide relative to the housing.

7. An instrument as claimed in claim 5, in which the shaft (120) has a connector (122) for a tool by which the shaft can be driven rotationally relative to the housing (4).

8. An instrument as claimed in claim 1, in which the ramp surface (30) is provided by the housing (4).

9. An instrument as claimed in claim 1, in which a ramp surface which is engaged by the follower is provided on at least one of the arms.

10. An instrument as claimed in claim 1, in which the ramp surface (30) defines a plurality of steps (32, 34, 36, 38) corresponding to predetermined positions of the shaft (120) relative to the housing (4).

11. An instrument as claimed in claim 1, which includes (a) a ratchet mechanism (127) which restricts movement of the follower relative to the ramp surface to movement in an engage direction, and (b) a release device (25) for disengaging the ratchet mechanism to allow movement in an opposite disengage direction.

12. An instrument as claimed in claim 11, in which each of the arms (62, 64) is biassed to slide outwardly relative to the housing (4).

## Patentansprüche

1. Instrument (2) zum Verwenden beim Manipulieren einer Implantatkomponente, die eine Seite (266) mit zwei, in einem Abstand angeordneten Kanten (268, 270) aufweist, wobei das Instrument Folgendes umfasst:
a. ein Gehäuse (4),
b. einen ersten und einen zweiten Schenkel (62, 64), wovon jeder einen Finger (76, 78), der die Implantatkomponente an einer jeweiligen Kante der Seite einkoppeln kann, aufweist, wobei die Schenkel für eine gleitende Bewegung relativ zu dem Gehäuse montiert sind, wobei sich die Finger auf den Schenkeln entlang einer gemeinsamen Achse bewegen,
c. einen Gangmechanismus (70, 124), durch welchen der erste und der zweite Schenkel miteinander verbunden sind, der gewährleistet, dass eine gleitende Bewegung des ersten Schenkels in eine erste Richtung relativ zu dem Gehäuse von einer gleichen gleitenden Bewegung des zweiten Schenkels in eine zweite Richtung, welche zu der ersten Richtung entgegengesetzt ist, begleitet ist, und
d. einen Schaft (120), der in dem Gehäuse montiert ist,
**dadurch gekennzeichnet, dass** der Schaft in dem Gehäuse montiert ist, so dass er entlang einer Schaftachse, die senkrecht zu der Achse, entlang welcher die Finger gleiten, ist, gleiten kann, wobei der Schaft an seinem Ende ein Auflager (46) zum Einkoppeln der Seite der Implantatkomponente aufweist,
wobei das Instrument eine Rampenfläche (30) und eine Tastnase (125), die sich entlang der Rampenfläche bewegen kann, aufweist, wobei die Rampenfläche oder die Tastnase relativ zu dem Schaft fixiert ist, so dass die gleitende Position des Schafts relativ zu dem Gehäuse entlang der Schaftachse durch die Position der Tastnase auf der Rampenfläche bestimmt ist, wobei die Position der Tastnase auf der Rampenfläche entsprechend den Positionen des ersten und des zweiten Schenkels relativ zu dem Gehäuse variiert.

2. Instrument gemäß Anspruch 1, bei dem die Tastnase (125) einen Vorsprung auf dem Schaft (120), der sich in eine Richtung im Allgemeinen transversal relativ zu der Schaftachse erstreckt, umfasst.

3. Instrument gemäß Anspruch 2, bei dem der Vorsprung einen Stift (125) umfasst.

4. Instrument gemäß Anspruch 1, bei dem der Gangmechanismus Folgendes umfasst:
eine erste und eine zweite Zahnstange (70), die an dem ersten bzw. dem zweiten Schenkel (62, 64) befestigt sind, und ein Zahnrad (124), das in dem Gehäuse (4) zwischen den Zahnstangen positioniert ist, dessen Zähne die Zähne auf der ersten und der zweiten Zahnstange einkoppeln, und das in dem Gehäuse zum Rotieren gebracht wird, wenn die Schenkel relativ zu dem Gehäuse gleiten.

5. Instrument gemäß Anspruch 1, bei dem der Gangmechanismus (124), der den ersten Schenkel (62) mit dem zweiten Schenkel (64) verbindet, bewirkt, dass sich der Schaft (120) um die Schaftachse dreht, wenn der erste Schenkel relativ zu dem zweiten Schenkel zum Gleiten gebracht wird.

6. Instrument gemäß Anspruch 5, bei dem der Gangmechanismus eine erste und eine zweite Zahnstange (70) umfasst, die an dem ersten bzw. dem zweiten Schenkel (62, 64) befestigt sind, und wobei der Schaft (120) ein Zahnrad (124), das sich zwischen den Zahnstangen befindet, aufweist, dessen Zähne die Zähne auf der ersten und der zweiten Zahnstange einkoppeln, so dass der Schaft in dem Gehäuse durch die Wirkung der Stangen auf das Zahnrad zum Drehen gebracht wird, wenn die Schenkel relativ zu dem Gehäuse gleiten.

7. Instrument gemäß Anspruch 5, bei dem der Schaft (120) ein Verbindungselement (122) für ein Werkzeug, durch welches der Schaft rotatorisch relativ zu dem Gehäuse (4) angetrieben werden kann, aufweist.

8. Instrument gemäß Anspruch 1, bei dem die Rampenfläche (30) von dem Gehäuse bereitgestellt ist.

9. Instrument gemäß Anspruch 1, bei dem eine Rampenfläche, die von der Tastnase eingekoppelt ist, auf zumindest einem der Schenkel bereitgestellt ist.

10. Instrument gemäß Anspruch 1, bei dem die Rampenfläche (30) eine Mehrzahl von Stufen (32, 34, 36, 38) definiert, die vorbestimmten Positionen des Schafts (120) relativ zu dem Gehäuse (4) entsprechen.

11. Instrument gemäß Anspruch 1, das Folgendes aufweist: (a) einen Ratschenmechanismus (127), der eine Bewegung der Tastnase relativ zu der Rampenfläche auf eine Bewegung in eine Kopplungsrichtung einschränkt, und (b) eine Freigebevorrichtung (25) zum Auskoppeln des Ratschenmechanismus, um eine Bewegung in eine entgegengesetzte Auskoppelrichtung zu ermöglichen.

12. Instrument gemäß Anspruch 11, bei dem jeder der Schenkel (62, 64) vorgespannt ist, um relativ zu dem Gehäuse (4) nach außen zu gleiten.

## Revendications

1. Instrument (2) pour l'utilisation pour manipuler un composant d'implant qui possède une face (266) avec deux bord espacés (268, 270), l'instrument comprenant :
a. un boîtier (4),
b. des premier et deuxième bras (62, 64) ayant chacun un doigt (76, 78) pouvant venir en prise avec le composant d'implant à des bords respectifs de la face, les bras étant installés en vue d'un mouvement de coulissement relativement au boîtier où les doigts sur les bras se déplacent le long d'un axe commun,
c. un mécanisme d'engrenage (70, 124) par lequel les premier et second bras sont liés l'un à l'autre, qui assure que le mouvement de coulissement du premier bras dans une première direction relativement au boîtier s'accompagne d'un mouvement de coulissement égal du second bras dans une seconde direction qui est opposée à la première direction, et
d. un arbre (120) qui est installé dans le boîtier, **caractérisé en ce que** l'arbre est installé dans le boîtier de telle sorte qu'il peut coulisser le long d'un axe d'arbre qui est perpendiculaire à l'axe le long duquel les doigts coulissent, l'arbre ayant un lit (46) à son extrémité pour la mise en prise avec la face du composant d'implant,
l'instrument incluant une surface formant rampe (30) et un suiveur (125) apte à se déplacer le long de la surface formant rampe, un parmi la surface de rampe et le suiveur étant fixe relativement à l'arbre de sorte que la position de coulissement de l'arbre relativement au boîtier le long de l'axe de l'arbre est déterminée par la position du suiveur sur la surface formant rampe, où la position du suiveur sur la surface formant rampe varie en accord avec les positions des premier et second bras relativement au boîtier.

2. Instrument selon la revendication 1, dans lequel le suiveur (125) comprend une projection sur l'arbre (120) qui s'étend dans une direction qui est généralement transversal relativement à l'axe de l'arbre.

3. Instrument selon la revendication 2, dans lequel la projection comprend une goupille (125).

4. Instrument selon la revendication 1, dans lequel le mécanisme d'engrenage comprend des première et deuxième crémaillères dentées (70) qui sont fixées aux premier et deuxième bras (62, 64) respectivement, et un engrenage dentée (124) positionné dans le boîtier (4) entre les crémaillères dentées dont les dents viennent en prise avec les dents des première et deuxième crémaillères et qui réalisé pour tourner dans le boîtier lorsque les bras coulissent relativement au boîtier.

5. Instrument selon la revendication 1, dans lequel le mécanisme d'engrenage (124) qui relie le premier bras (62) au deuxième bras (64) amène l'arbre (120) à tourner autour de l'axe d'arbre lorsque le premier bras est amené à coulisser relativement au second bras.

6. Instrument selon la revendication 5, dans lequel le mécanisme d'engrenage comprend des première et deuxième crémaillères dentées (70) qui sont fixées aux premier et second bras (62, 64) respectivement, et dans lequel l'arbre (120) possède un engrenage dentée (124) situé entre les crémaillères dentées dont les dents viennent en prise avec les dents sur les première et deuxième crémaillères de sorte que l'arbre est amené à tourner dans le boîtier sous l'action des crémaillères sur l'engrenage dentée lorsque les bras coulissent relativement au boîtier.

7. Instrument selon la revendication 5, dans lequel l'arbre (120) possède un connecteur (122) pour un outil par lequel l'arbre peut être entraîné en rotation relativement au boîtier (4).

8. Instrument selon la revendication 1, dans lequel la surface formant rampe (30) est réalisée par le boîtier (4).

9. Instrument selon la revendication 1, dans lequel une surface formant rampe dans laquelle s'engage le suiveur est réalisé sur au moins un des bras.

10. Instrument selon la revendication 1, dans lequel la surface formant rampe (30) définit une pluralité de gradins (32, 34, 36, 38) correspondant à des positions prédéterminées de l'arbre (120) relativement au boîtier (4).

11. Instrument selon la revendication 1, qui comprend (a) un mécanisme de rochet (127) qui restreint le mouvement du suiveur relativement à la surface formant rampe en mouvement dans une direction d'engagement, et (b) un dispositif de libération (25) pour sortir de prise le mécanisme formant rochet pour permettre un déplacement dans une direction de désengagement opposée.

12. Instrument selon la revendication 11, dans lequel chacun des bras (62, 64) est sollicité pour coulisser vers l'extérieur relativement au boîtier (4).
